# EUROPÄISCHE PATENTSCHRIFT

(11) **EP 1 009 465 B1**
(45) Veröffentlichungstag und Bekanntmachung des Hinweises auf die Patenterteilung: **26.11.2003**
(21) Anmeldenummer: 98948793.9
(22) Anmeldetag: 08.08.1998
(51) Int. Cl.: A61M 16/04

(54) **TRACHEOTOMIEKANÜLE MIT SCHILD**
TRACHEOTOMY CANNULA WITH SHIELD
CANULE DE TRACHEOTOMIE AVEC ECRAN

(30) Priorität: 06.09.1997 DE 19739103
(43) Veröffentlichungstag der Anmeldung: 21.06.2000
(73) Patentinhaber: Tracoe Gesellschaft für Medizinische Bedarfsgegenstände MBH, 63263 Neu-Isenburg (DE)
(72) Erfinder: WALDECK, Franz, D-55268 Nieder-Olm (DE)
(74) Vertreter: Weber, Dieter, Dr. Dipl.-Chem.
(86) Internationale Anmeldenummer: DE9802344
(87) Internationale Veröffentlichungsnummer: WO99012599

(56) Entgegenhaltungen:
- WO-A-94/12231
- WO-A-96/11029
- DE-A- 19 510 783
- FR-A- 1 426 926
- US-A- 4 270 529
- US-A- 5 398 679

## Beschreibung

Die vorliegende Erfindung betrifft eine Tracheotomiekanüle, bestehend aus einem rohr- oder schlauchförmigen Element für die Hindurchführung durch einen Hals- und Luftröhrenschnitt in die Luftröhre eines Patienten, wenn dessen obere Atemwege blockiert sind, um den Patienten durch die Kanüle mit Atemluft zu versorgen, und mit einem an der Kanüle angebrachten Schild, durch dessen Anlage am Hals des Patienten die Position der Kanüle bzw. des Endes der Kanüle in der Luftröhre des Patienten definiert wird.

Eine solche Tracheotomiekanüle ist aus der DE 195 10 783 A1 bekannt.

Derartige Tracheotomiekanülen werden zum einen in der Chirurgie, d.h. während Operationen zum Beispiel im Bereich der oberen Atemwege, eingesetzt, zum anderen aber auch in der Intensivmedizin und schließlich auch für Patienten, deren obere Atemwege aufgrund einer Krankheit oder eines Unfalls auf Dauer blockiert sind und welche durch eine solche Kanüle atmen.

Insbesondere wenn derartige Kanülen für einen längeren Gebrauch gedacht sind, also zum Beispiel in der Intensivmedizin oder für einen Patienten, der dauerhaft mit einer solchen Kanüle atmen muß, ist ein gewisser Tragekomfort für den Patienten von erheblicher Bedeutung, um zum Beispiel ein Druckgefühl, Druckstellen oder Wundscheuern zu vermeiden. Der Position des Schildes an einer Tracheotomiekanüle kommt dabei insofern Bedeutung zu, als der Schild dazu dient, die Endposition der Tracheotomiekanüle an ihrem in die Luftröhre eingeschobenen Zustand zu definieren. Dabei verläuft ein unterer Abschnitt der Tracheotomiekanüle zunächst im wesentlichen senkrecht entlang der Luftröhre, und dann in einer relativ sanften Biegung um etwa 90° durch den Luftröhrenschnitt und den Halsschnitt des Patienten nach außen. Es versteht sich, daß die anatomischen Gegebenheiten bei den jeweiligen Patienten sehr verschieden sein können, insbesondere also der Luftröhrendurchmesser und der Abstand der Luftröhre von der Hautoberfläche des Halses, ebenso wie auch die Lage der Schnitte im Hals und in der Luftröhre.

Um den unterschiedlichen anatomischen Gegebenheiten Rechnung zu tragen, gibt es bereits Tracheotomiekanülen in den verschiedensten Abmessungen und Größen. Dabei variiert insbesondere der Durchmesser der Tracheotomiekanülen, in gewissem Rahmen aber auch ihre Länge und ihr Krümmungsradius in der Biegung, die den Übergang von dem vertikalen, in die Luftröhre hineinragenden Abschnitt zu dem außen aus dem Hals des Patienten vorstehenden Abschnitt bildet. Im Bereich dieser Biegung oder am Ende der Biegung sitzt der Schild, wobei
die Tracheotomiekanüle grundsätzlich so weit in den Hals und die Luftröhre eines Patienten eingeführt wird, daß der Schild an dem Hals des Patienten anliegt. Wenn jedoch die Tracheotomiekanüle im wesentlichen nach ihrem Außendurchmesser für einen Patienten passend ausgewählt wird, so bedeutet dies noch nicht, daß auch die übrigen anatomischen Gegebenheiten des Patienten mit den durch die Lage des Schildes festgelegten Maßen übereinstimmen. Es kann also durchaus sein, daß der Abstand der Luftröhre des Patienten zur Halsoberfläche größer oder kleiner ist als der Abstand des Schildes der Tracheotomiekanüle von dem vertikalen Abschnitt der Tracheotomiekanüle bzw. dessen gedachter Verlängerung. In diesem Fall würde der Patient unangenehme Druck- oder Zugkräfte spüren, wenn die Tracheotomiekanüle bis zur Anlage des Schildes an die Halsoberfläche eingeschoben wird.

Zwar ist es grundsätzlich auch möglich, Tracheotomiekanülen nicht nur mit verschiedenen Durchmessern, sondern auch mit unterschiedlichen Schildabständen zu dem vertikalen Abschnitt bereitzuhalten, jedoch macht dies die Lagerhaltung und auch das jeweilige Auswählen und Anpassen einer Tracheotomiekanüle für einen Patienten relativ aufwendig und teuer.

Ausgehend von diesem Stand der Technik liegt der vorliegenden Erfindung die Aufgabe zugrunde, eine Tracheotomiekanüle mit den eingangs genannten Merkmalen zu schaffen, welche in einfacher Weise an die bei einem Patienten vorliegenden, anatomischen Gegebenheiten anpaßbar ist und keinen größeren Aufwand hinsichtlich der Bevorratung und Anpassung von Tracheotomiekanülen verursacht.

Diese Aufgabe wird dadurch gelöst, daß der Schild der Tracheotomiekanüle in Längsrichtung der Kanüle in verschiedenen Positionen fixierbar an dieser angebracht ist, wobei der Schild an einer Klemmvorrichtung angebracht ist, die mit der Außenseite der Kanüle in lösbarem Klemmeingriff steht und die Klemmvorrichtung in Klemmeingriff mit der Kanüle vorgespannt ist.

In diesem Fall braucht die Tracheotomiekanüle lediglich hinsichtlich eines geeigneten Durchmessers ausgewählt werden und wird dann durch den Hals- und Luftröhrenschnitt in Hals und Luftröhre des Patienten eingeführt. Dies kann gegebenenfalls auch unter Röntgenkontrolle erfolgen. Wenn dann die Kanüle eine Position eingenommen hat, die sowohl hinsichtlich ihrer Funktionsfähigkeit geeignet ist als auch anatomisch korrekt ist, indem sie an keiner Stelle einseitige Druck- oder Zugkräfte auf das umgebende Gewebe ausübt, wird der zunächst in eine weitestmögliche Stellung zurückgezogene Schild auf der Tracheotomiekanüle in einer Position fixiert, in welcher er am Hals des Patienten anliegt, ohne daß die Kanüle dabei weiter in den Halsschnitt hineingeschoben oder herausgezogen wird.

Die Klemmvorrichtung hat den Vorteil, daß sie stufenlos an der Tracheotomiekanüle befestigbar ist und daß außerdem an der Kanüle selbst keinerlei Vorrichtungen, Profilierungen oder Raststellen vorgesehen werden müssen, sondern daß die Kanüle ein im wesentlichen glattes Rohr sein kann, auf welchem die Klemmvorrichtung in geeignete Positionen verschoben und dann an der Außenfläche der Kanüle festgeklemmt wird.

Die Klemmvorrichtung ist, solange sie nicht aktiv betätigt wird, in Klemmeingriff vorgespannt. Dies bedeutet, daß der an der Klemmvorrichtung befestigte Schild die einmal eingenommene Position an der Tracheotomiekanüle behält und nur bei Betätigung, d. h. bei Lösen des vorgespannten Klemmeingriffes, auf der Kanüle verschiebbar ist.

Besonders bevorzugt ist eine Ausführungsform der Erfindung, bei welcher die Klemmvorrichtung aus mindestens zwei Halteteilen besteht, welche jeweils eine im wesentlichen kreisförmige Öffnung aufweisen, durch welche die Tracheotomiekanüle hindurchgeführt ist, wobei die beiden, die Kanüle umgreifenden Halteteile relativ zueinander in Richtung senkrecht zu den Achsen der Öffnungen bzw. senkrecht zur Achse der Kanüle verschiebbar sind. Dies bedeutet, daß die beiden Öffnungen, wenn sie zum Beispiel in Flucht miteinander gebracht werden, ohne weiteres gemeinsam die Kanüle aufnehmen können und auf der Außenfläche der Kanüle verschiebbar sind, wohingegen nach dem relativen Verschieben senkrecht zur Achse der Kanüle bzw. senkrecht zur Achse der Öffnungen, diese Öffnungen einander noch teilweise überlappen und in dem Überlappungsbereich die Kanüle festklemmen.

Gemäß einer Ausführungsform der Erfindung ist vorgesehen, daß die mindestens zwei, die Öffnungen aufweisenden Halteteile um eine dritte Achse verschwenkbar sind, die ebenfalls parallel zu den Achsen der beiden Öffnungen verläuft, jedoch nicht mit einer dieser Achsen zusammenfällt. Werden die beiden Teile relativ zueinander um diese dritte Achse verschwenkt, so ändert sich wiederum der Überlappungsquerschnitt der beiden Öffnungen, so daß dadurch der gewünschte Klemmeffekt erzeugt werden kann.

In einer anderen Variante ist das eine Halteteil relativ zu dem anderen im wesentlichen linear verschiebbar, und zwar wiederum senkrecht zur Achse der jeweiligen Öffnungen bzw. zur Achse der Kanüle. Besonders bevorzugt ist eine Ausführungsform der Erfindung, bei weicher die Klemmvorrichtung im wesentlichen aus drei Teilen besteht, wobei zwei der Teile fest miteinander verbunden sind (alternativ auch einstückig zusammenhängen könnten) und gemeinsam ein Gehäuse bilden, welches zwei miteinander fluchtende Öffnungen für das Hindurchführen einer Tracheotomiekanüle aufweist, und wobei ein drittes, im wesentlichen ebenes Teil vorgesehen ist, das in dem Gehäuse aufgenommen ist und ebenfalls eine Öffnung für die Hindurchführung einer Tracheotomiekanüle hat, weiche mindestens teilweise mit den beiden miteinander fluchtenden Öffnungen der ersten und zweiten Teile in Überlappung bringbar ist, so daß die Tracheotomiekanüle alle drei Öffnungen durchgreifen kann, wobei der dritte, ebene Teil in dem Gehäuse derart vorgespannt ist, daß seine Öffnung in eine zu den beiden anderen Öffnungen exzentrische Lage verschoben wird, wodurch die Tracheotomiekanüle in dem überlappenden Teil der drei Öffnungen eingeklemmt wird.

Die beiden, das Gehäuse bildenden Teile können zum Beispiel jeweils einen Schnapprand aufweisen und zusammengesteckt werden, während das dritte, im wesentlichen ebene Teil dazwischen bzw. in einem der Gehäuseteile aufgenommen ist. Die beiden Gehäuseteile können auch, zum Beispiel durch ein Biegescharnier, einstückig miteinander verbunden sein. Die Mehrteiligkeit des Gehäuses dient vor allem dem Zweck, das dritte, ebene Teil sicher in dem aus den beiden anderen Teilen gebildeten Gehäuse aufzunehmen, ohne daß es sich aus dem Gehäuse herausbewegen kann.

Zur Erzeugung der exzentrischen Vorspannung weist das ebene Teil vorzugsweise einen Federbügel auf, der sich an der Innenwand des von dem ersten und zweiten Teil gebildeten Gehäuses abstützt. Der Federbügel kann zum Beispiel aus einem entlang eines Kreisbogens gebogenen Bügel bestehen, der mit einem mittleren Abschnitt an einem Ende des ebenen Teiles befestigt ist bzw. einstückig mit diesem verbunden ist und zwei seitliche und nach vorn wegstehende, elastisch wegbiegbare Schenkel aufweist. Selbstverständlich kann der Federbügel auch ein separates Teil sein und auch als ein andersartiges Federelement ausgebildet sein.

Auf der dem Federbügel gegenüberliegenden Seite weist das ebene Teil vorzugsweise ein Betätigungselement auf, welches sich durch eine in dem Gehäuse freigelassene seitliche Öffnung hindurch nach außen erstreckt, so daß das Betätigungselement von der Gehäuseaußenseite her gegen die von dem Federbügel ausgeübte Kraft eindrückbar ist, wobei die drei Teile relativ zueinander so angeordnet sind, daß sich beim Eindrücken des Betätigungselementes der Überlappungsbereich der drei Öffnungen vergrößert und gegebenenfalls die drei Öffnungen auch vollständig zur Deckung gebracht werden können, so daß in diesem Zustand die Tracheotomiekanüle, deren Außendurchmesser geringfügig geringer ist als der Innendurchmesser der drei Öffnungen, leicht in den drei miteinander fluchtenden Öffnungen verschoben werden kann, bzw. umgekehrt die Klemmvorrichtung auf der Tracheotomiekanüle leicht verschoben werden kann. Sobald jedoch das Betätigungselement losgelassen wird, drückt der Federbügel die mittlere der zwei Öffnungen, die sich an dem in dem Gehäuse befindlichen ebenen Teil befindet, in eine exzentrische Lage, so daß hierdurch die Tracheotomiekanüle eingeklemmt wird.

Zweckmäßigerweise ist der Innenrand der Öffnungen einer solchen Vorrichtung gezahnt oder gezackt ausgebildet, damit die Klemmvorrichtung einen festen Sitz auf der Tracheotomiekanüle behält.

An der Klemmvorrichtung ist, wie bereits erwähnt, der Schild der Tracheotomiekanüle befestigt, so daß mit der Klemmvorrichtung auch der Schild bewegbar ist. Zweckmäßigerweise ist der Schild an der dem Hals des Patienten zugewandten Seite der Klemmvorrichtung angebracht. Eine Betätigungsvorrichtung zum Lösen der Klemmvorrichtung, um diese zusammen mit dem Schild entlang der Tracheotomiekanüle verschieben zu können, befindet sich dementsprechend auf der dem Hals eines Patienten abgewandten Seite des Schildes an der Klemmvorrichtung.

Die Klemmvorrichtung, die mit dem Schild der Tracheotomiekanüle verbunden ist, ist in der bevorzugten Ausführungsform der Erfindung so gestaltet, daß sie mit den Fingern einer Hand entsperrt und auf der Tracheotomiekanüle verschoben werden kann. Diese Eigenschaft weisen auch die nachstehend im Detail näher beschriebenen Ausführungsformen auf. Konkret wird dies dadurch erreicht, daß zum einen, wie bereits erwähnt, die Klemmvorrichtung mit dem Schild verbunden ist und zum anderen dadurch, daß die Klemmvorrichtung aus mindestens zwei relativ zueinander federnd vorgespannten Teilen besteht, die in Sperrichtung aufeinander vorgespannt sind, so daß sie sich an der Außenwand einer Tracheotomiekanäle festklemmen können und die so zusammenhängend ausgebildet und zusammenmontiert sind, daß zum Beispiel eines der Teile der Klemmvorrichtung gegenüber anderen Teilen vorsteht, so daß die beiden Teile mit den Fingern einer Hand ergriffen und der vorstehende Teil zurückgedrückt werden kann, wobei die Entsperrung der in Richtung aufeinander vorgespannten Teile erfolgt. Bei der Handhabung eines solchen Tracheotomietubus ist es oftmals von großer Bedeutung, daß entweder der Patient selbst oder aber ein Arzt oder eine Pflegeperson bei Verschieben des Schildes eine Hand frei behält, um damit irgendein anderes Instrument betätigen oder halten zu können, so daß es von großem Vorteil ist, wenn die Klemmvorrichtung mit nur einer einzigen Hand betätigt und gemeinsam mit dem Schild auf dem Tracheotomietubus verschoben werden kann.

In der bevorzugten Ausführungsform der Erfindung weist der Schild eine Öffnung auf, deren Durchmesser größer ist als der Durchmesser der Tracheotomiekanüle und auch größer als der Durchmesser der Öffnungen der Klemmvorrichtung, und der Schild ist mit seiner, vorzugsweise kreisförmigen, Öffnung auf einen zylindrischen Ansatz der Klemmvorrichtung aufgesetzt, der zu einer angrenzenden Öffnung der Klemmvorrichtung für die Hindurchführung der Tracheotomiekanüle im wesentlichen konzentrisch angeordnet ist.

Der zylindrische Ansatz, der den Schild trägt, weist an seinem Ende vorzugsweise einen radial nach außen ragenden Halteflansch auf.

Weiterhin besteht in der bevorzugten Ausführungsform der Erfindung der Schild aus zwei getrennten, relativ zueinander beweglichen Flügeln, die jeweils einen Ringabschnitt mit einer Öffnung aufweisen, der auf den zylindrischen Ansatz aufgesetzt ist.

Dabei weisen die Ringabschnitte auf ihren einander zugewandten Flächen vorzugsweise eine Riffelung oder Verzahnung auf, welche ein relatives Verdrehen der Ringabschnitte und damit der den Schild bildenden Flügel behindern. Allerdings soll das relative Verdrehen unter Überwindung einer entsprechenden Widerstandskraft immer noch möglich sein, so daß sich die durch die Riffelung bzw. Verzahnung vorgegebenen unterschiedlichen relativen Positionen der Flügel im wesentlichen fest einstellen lassen.

Die Ringabschnitte sind dabei zwischen dem Halteflansch des zylindrischen Ansatzes und dem Grund des zylindrischen Ansatzes, d.h. der Oberfläche des Teiles, von welchem der zylindrische Ansatz ausgeht, in Eingriff miteinander mehr oder weniger vorgespannt und werden jedenfalls so zusammengehalten, daß sie nur unter Überwindung gewisser Haltekräfte gegeneinander verdrehbar sind. Dabei kann eventuell einer der Flügel auch so an der Klemmvorrichtung bzw. dem zylindrischen Ansatz befestigt sein, daß er gegenüber der Klemmvorrichtung nicht drehbar ist, so daß nur der andere Flügel relativ zu dem erstgenannten und der Klemmvorrichtung drehbar ist und die Position des erstgenannten Flügels zusammen mit der Klemmvorrichtung in der gewünschten Lage eingestellt wird.

Weitere Vorteile, Merkmale und Anwendungsmöglichkeiten der Erfindung ergeben sich aus der folgenden Beschreibung einer bevorzugten Ausführungsform und der dazugehörigen Figuren.
Es zeigen:
- Figur 1: eine perspektivische Ansicht einer Tracheotomiekanüle mit beweglichem Schild,
- Figur 2: verschiedene Ansichten der erfindungsgemäßen Klemmvorrichtung,
- Figur 3: das erste Gehäuseteil der Klemmvorrichtung aus Figur 2 in verschiedenen Ansichten,
- Figur 4: das zweite Gehäuseteil der Klemmvorrichtung aus Figur 2 in verschiedenen Ansichten,
- Figur 5: das dritte, ebene Teil der Klemmvorrichtung nach Figur 2 und
- Figur 6: einen von zwei einen Schild bildenden Flügel in verschiedenen Ansichten.

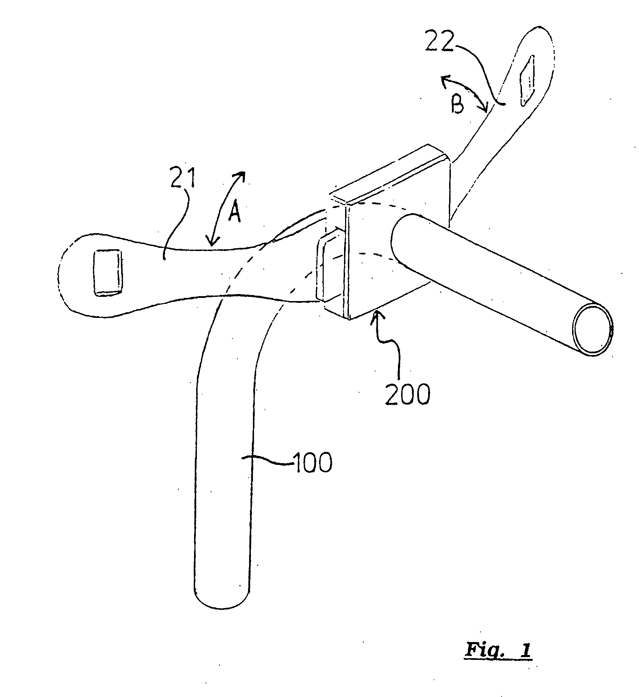

Man erkennt in Figur 1 eine Tracheotomiekanüle 100 mit einem Schild in Form zweier Flügel 21, 22, die an einer Klemmvorrichtung 200 befestigt sind. Die Klemmvorrichtung 200 ist zusammen mit den Flügeln 21, 22 in Längsrichtung auf der Tracheotomiekanüle bewegbar, so daß der Schild 21, 22 in eine beliebige, für einen Patienten geeignete Position eingestellt werden kann. Außerdem sind die Flügel 21, 22 noch um die Achse der Tracheotomiekanüle 100 verschwenkbar, was durch die Pfeile A, B angedeutet wird.

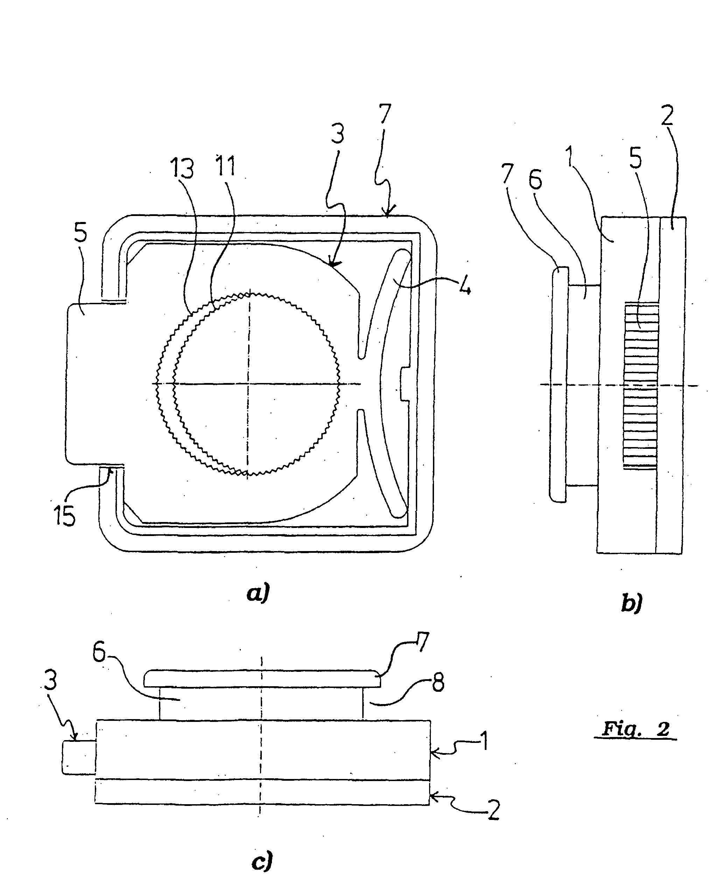

Die Figuren 2 bis 5 zeigen weitere Einzelheiten der Klemmvorrichtung 200.

Die Figuren 2b und 2c zeigen die Klemmvorrichtung 200 in zwei verschiedenen, um 90° zueinander versetzten Seitenansichten, während Figur 2a die Klemmvorrichtung in einer Ansicht zeigt, die der Blickrichtung in Figur 2b von unten gemäß den Pfeilen IIa, IIa entspricht, wobei jedoch das untere Gehäuseteil 2 fortgelassen ist.

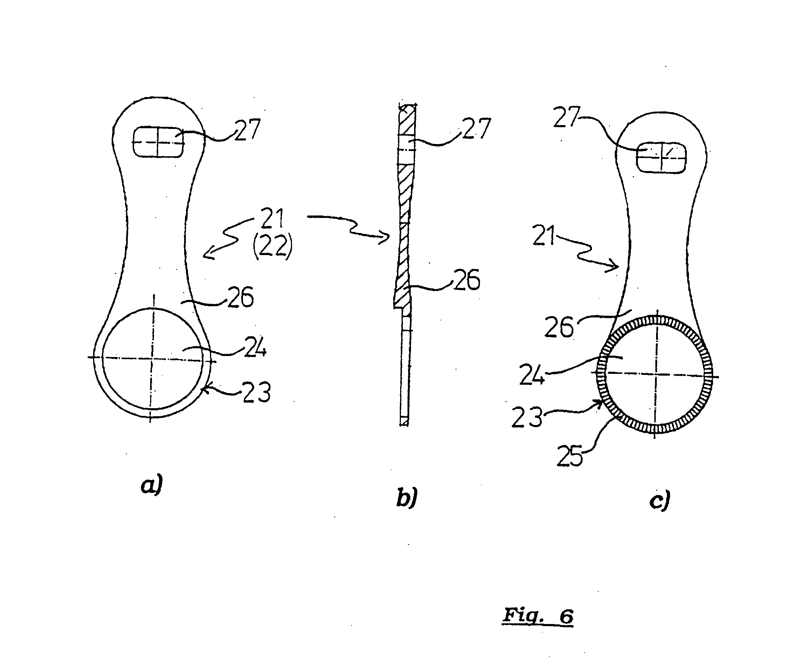

Wie man anhand der Figuren 2b und 2c erkennen kann, besteht die Klemmvorrichtung 200 im wesentlichen aus zwei zusammengesteckten Gehäuseteilen 1, 2 und einem dazwischen in das Gehäuse eingelegten ebenen Teil 3. Das Gehäuseteil 1 weist noch einen zylindrischen Ansatz 6 mit einem Halteflansch 7 auf, so daß in dem Zwischenraum 8, der zwischen dem Halteflansch 7 und der an den zylindrischen Ansatz 6 angrenzenden Oberfläche des Gehäuseteiles 1 gebildet wird, Ringelemente 23 aufgenommen werden, die im Zusammenhang mit Figur 6 beschrieben werden.

In Figur 2a erkennt man in der Draufsicht das in das Gehäuseteil 1 eingelegte ebene Teil 3. Das Gehäuseteil 1 weist in seiner, durch das ebene Teil 3 teilweise verdeckten oberen Wand eine in etwa kreisförmige Öffnung 11 auf, während das ebene Teil 3 eine entsprechende kreisförmige Öffnung 13 mit demselben Durchmesser aufweist, die jedoch in der dargestellten Position, welche der Ruheposition der Klemmvorrichtung 200 entspricht, exzentrisch zu der Öffnung 11 verschoben ist. Das untere Gehäuseteil 2 weist eine entsprechende, kreisförmige Öffnung 12 auf, die im zusammenmontierten Zustand mit der Öffnung 11 des oberen Gehäuseteils 1 fluchtet.

Die in Figur 2a erkennbare, exzentrische Position der Öffnung 13 gegenüber der Öffnung 11 wird durch einen Federbügel 7 hervorgerufen, der an einem Ende des ebenen Teils 3 einstückig angebracht ist und der sich mit seinen beiden nach außen und vorn erstreckenden Flügeln an der Innenwand des Gehäuseteiles 1 abstützt. Auf der dem Federbügel 7 entgegengesetzten Seite des ebenen Teiles 3 befindet sich eine Betätigungslasche bzw. ein Betätigungszapfen 5, der sich durch eine seitliche Öffnung 15, die durch eine Aussparung in der Seitenwand des Gehäuseteiles 1 gebildet wird, hindurch erstreckt. Wird dieser Betätigungszapfen 5 in die Öffnung 15 hineingedrückt, so gibt der Federbügel 4 elastisch nach und die beiden Öffnungen 11, 13 können im wesentlichen zur Deckung gebracht werden. Ein seitliches Ausweichen des ebenen Teiles 3 wird zum einen durch eine entsprechend enge Passung des Zapfens 5 in der Aussparung 15 und im übrigen auch durch die im wesentlichen passende Aufnahme des ebenen Teiles 3 zwischen den Seitenwänden des Gehäuseteiles 1 verhindert. In der Ansicht gemäß Figur 2a kann daher das ebene Teil 3 in dem Gehäuseteil 1 im wesentlichen nur lineare seitliche Bewegungen ausführen, wobei der Federbügel 4 mehr oder weniger vorgespannt wird.

Wenn der Zapfen 5 nicht eingedrückt wird, sorgt die Federspannung des Federbügels 4 dafür, daß die Tracheotomiekanüle, die sich gleichzeitig durch die Öffnungen 11, 13 hindurch erstreckt und einen geringfügig kleineren äußeren Durchmesser hat als die Öffnungen 11, 13, von den gezahnten Innenflächen der Öffnungen 11, 13 eingeklemmt wird, wodurch die Klemmvorrichtung 200 sich an der Außenwand der Tracheotomiekanüle 100 im wesentlichen unverrückbar festhält.

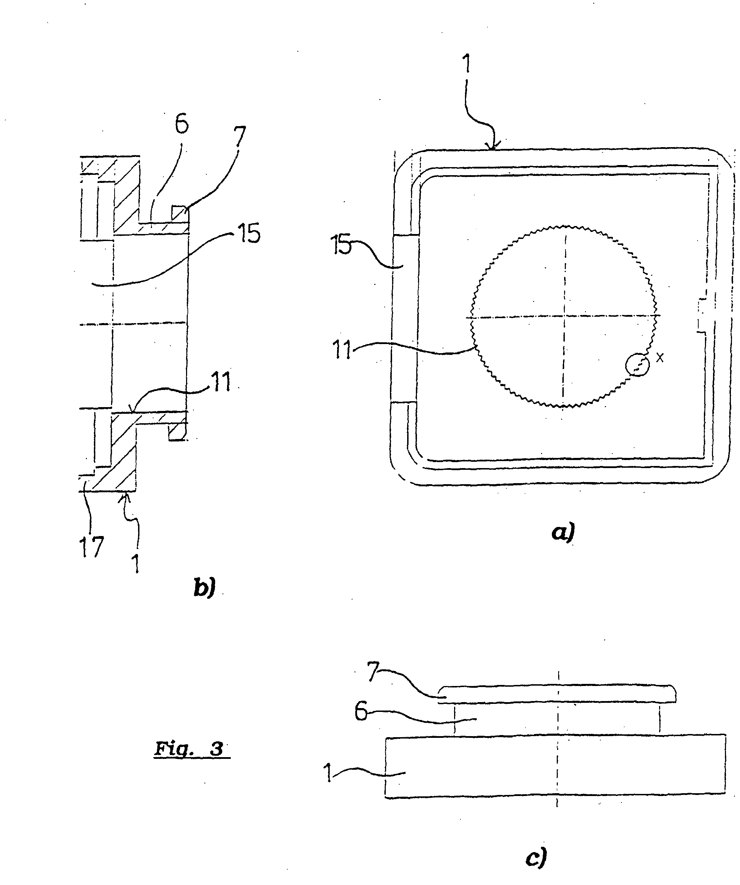

Die Figuren 3 bis 5 zeigen die Einzelteile, aus welchen die Klemmvorrichtung 200 aufgebaut ist, im einzelnen, jeweils in verschiedenen Ansichten.

In Figur 3 erkennt man das Gehäuseoberteil 1. Dies besteht im wesentlichen aus einem flachen, rechteckigen Quader mit abgerundeten Ecken, dessen eine Seitenwand die bereits erwähnte Aussparung 15 für die Hindurchführung des Betätigungszapfens 5 aufweist und dessen obere Wand, welche die Öffnung 11 aufweist, mit einem zylindrischen Ansatz 6 versehen ist, an dessen freien Ende ein Halteflansch 7 sich radial nach außen erstreckt, wobei der zylindrische Ansatz 6 konzentrisch zu der Öffnung 11 verläuft.

Die Seitenwände des Gehäuseteiles 1 sind, wie man in der Schnittdarstellung gemäß Figur 3b erkennt, abgestuft ausgebildet, so daß ein entsprechend abgestufter Ansatz 17 des Gehäuseteiles 2 passend mit den Seitenwänden des Gehäuseteiles 1 zusammengesteckt werden kann. Figur 3c ist eine Ansicht auf das Gehäuseteil 1, welche der Blickrichtung von unten auf das in Figur 2a dargestellte Gehäuseteil 1 entspricht.

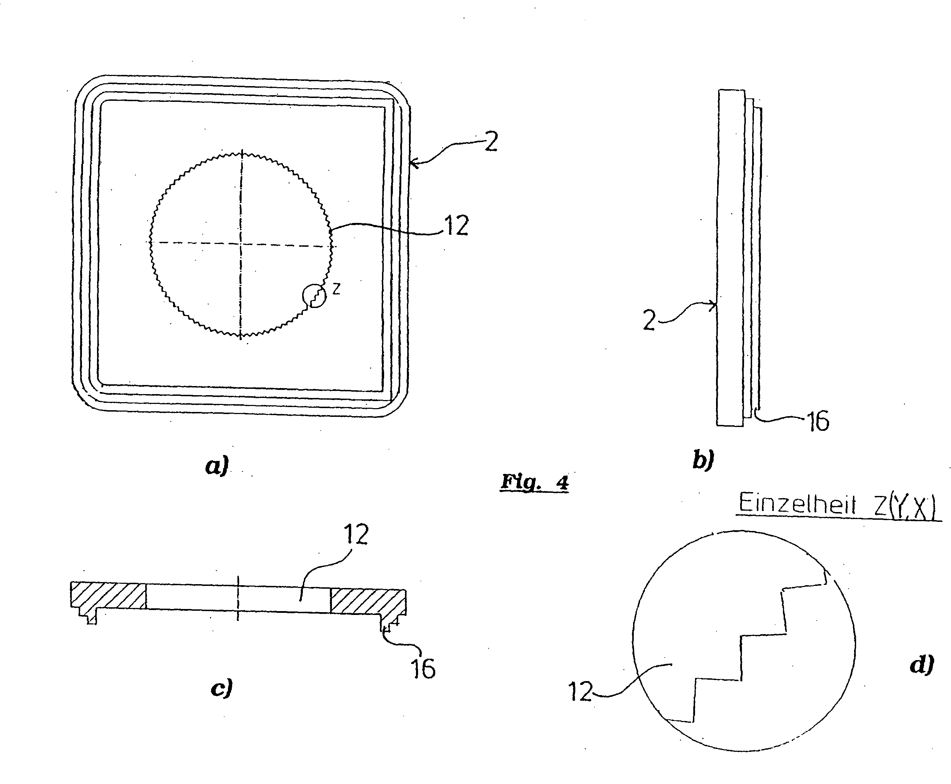

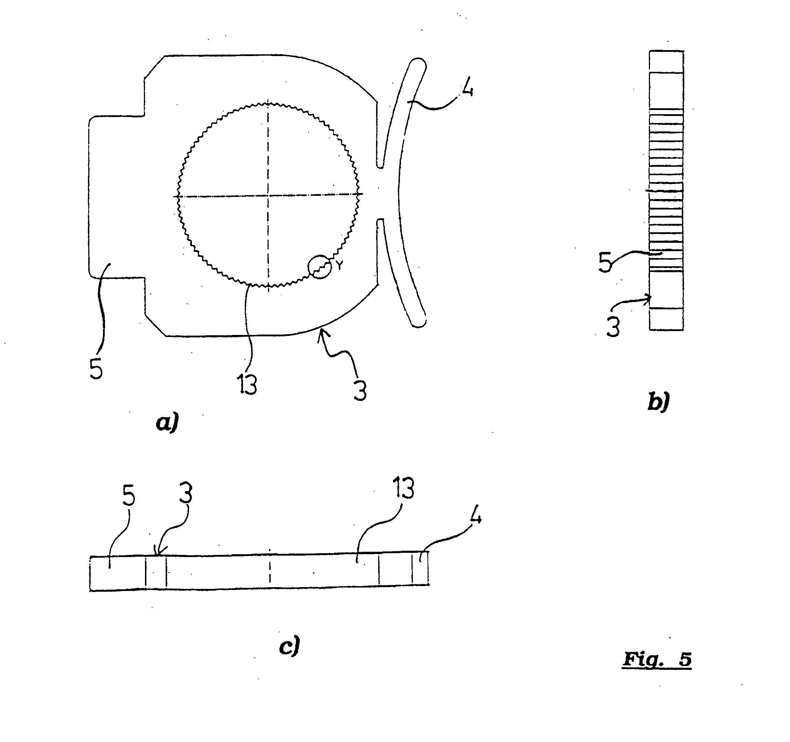

In Figur 4a ist das Gehäuseunterteil 2 in einer Ansicht vom Inneren des Gehäuses her dargestellt, Figur 4b zeigt einen vertikalen Schnitt durch das Zentrum des Gehäuseunterteiles 2 und Figur 4c zeigt eine Seitenansicht, in welcher man den abgestuften Ansatz 16 erkennt, dessen Außendurchmesser auf den Innendurchmesser des komplementären Ansatzes 17 am Gehäuseteil 1 abgestimmt ist, so daß die beiden Gehäuseteile in enger Passung, nach Möglichkeit in Preßpassung, zusammengesteckt bzw. zusammengedrückt werden können, so daß sie in Form des in Figur 2 dargestellten Gehäuses mit dem darin aufgenommenen ebenen Teil zusammenhalten. Figur 4d zeigt einen Ausschnitt aus dem Rand der Öffnung 12, wobei dies auch genau den Einzelheiten Y und X aus den Figuren 3a und 5a entspricht. Der gezackte Rand der Öffnung 12 sorgt für einen festen unverrückbaren Eingriff der Klemmvorrichtung auf der Tracheotomiekanüle 100.

In Figur 5 erkennt man das in dem Gehäuse, welches aus den Teilen 1 und 2 gebildet wird, aufgenommene ebene Teil 3. Dieses weist eine Öffnung 13 mit genau demselben Durchmesser wie die Öffnungen 11 und 12 auf, wird jedoch durch den Federbügel 4 in dem Gehäuse exzentrisch zu den genannten Öffnungen 11 und 12 vorgespannt. Der flache Betätigungszapfen 5 dient dazu, die Öffnungen 11, 12, 13 zur Deckung zu bringen, indem der mit seinen Enden an der Gehäuseinnenwand anliegende Federbügel 4 aus seiner normalerweise gekrümmten Stellung flachgedrückt wird.

Figur 6 zeigt einen der beiden Flügel 21, 22, die zusammen den Schild bilden, wobei diese beiden Flügel 21, 22 identisch sind, so daß nur einer dieser Flügel hier gezeigt zu werden braucht. Figur 6a zeigt eine rückwärtige Ansicht eines Flügels 21, 22, Figur 6b zeigt den Flügel im Längsschnitt und Figur 6c zeigt eine Draufsicht von vorn.

Der Flügel 21, 22 ist ein längliches, in der Draufsicht in etwa knochenförmiges Teil mit verbreiterten Enden und einem schmaleren Zwischenteil, wobei der Flügel 21 bzw 22 jedoch, wie man anhand der Figuren 6b und 6d erkennt, relativ flach und dünn ausgebildet ist. Das eine Ende ist mit einer zentralen Öffnung 24 versehen, deren Durchmesser dem Außendurchmesser des zylindrischen Ansatzes 6 des Halteteiles 1 der Klemmvorrichtung entspricht. Diese Öffnung 24 wird gebildet bzw. ist umgeben von einem Ringelement 23, dessen eine Seite mit einer Verzahnung bzw. Riffelung 25 versehen ist, die man am besten in Figur 6c erkennt. Die Zahnkämme dieser Verzahnung 25 verlaufen dabei im wesentlichen radial.

Wie man außerdem anhand der Figur 6b erkennt, ist der Ringabschnitt 23 des Flügels 21 bzw. 22 nur halb so dick ausgebildet wie der angrenzende Teil des Flügels 21, 22. Dabei ist die Verzahnung bzw. Riffelung 25 auf der in Figur 6b linken Seite des Ringelementes 23 vorgesehen. Wie man sich leicht vorstellen kann, können dann zwei derartige Flügel 21, 22, die zum Beispiel in entgegengesetzte Richtung weisen, mit ihren Ringelementen passend aufeinandergelegt werden, wobei die Verzahnungen 25 der Ringelemente 23 einander zugewandt sind und miteinander in Eingriff treten. Wegen des abgestuften Überganges von dem Ringelement 23 zu dem angrenzenden, doppelt so dicken Abschnitt 26 des Flügels 21, 22 hat dann der aus den beiden Flügeln 21, 22 zusammengesetzte Schild in diesem Bereich eine durchgehend konstante Dicke.

Da sich der Übergang von dem dickeren Abschnitt 26 des Flügels zu dem Ringelement 23 nur über einen Winkelbereich von etwa 90 bis 100° erstreckt, können die beiden mit ihren Ringelementen 23 passend zusammengelegten Flügel 21, 22 aus ihrer Lage, in welcher die beiden Flügel in entgegengesetzte Richtungen weisen, noch relativ zueinander um die gemeinsame Achse der Ringelemente verschwenkt werden, so daß sie einen Winkel von minimal 100° und maximal 180° miteinander einschließen können. Es versteht sich, daß die Flügel 21, 22 ohne weiteres auch so ausgestaltet werden können, daß sie über einen größeren, beliebigen Winkelbereich, zum Beispiel von 0° bis 180°, gegeneinander verschwenkt werden können.

Die beiden Ringelemente 23 werden auf den zylindrischen Ansatz 6 des Gehäuseteiles 1 aufgesetzt, wobei die Ringelemente genügend dehnbar sind (wahlweise auch der Flanschabschnitt 7 entsprechend zusammendrückbar bzw. verformbar ist), um über den Flanschabschnitt hinweg bewegt werden zu können. Der Flanschabschnitt 7 hält dann die beiden Ringelemente 23 so zusammen, daß die Verzahnungen 25 in genügend festem Eingriff bleiben, um die beiden Flügel 21, 22 in einer gewählten Winkelstellung zu halten; wobei jedoch diese Flügel unter Aufbringung einer gewissen Kraft noch ohne größeren Aufwand gegeneinander verdrehbar sind, so daß sie relativ zueinander eine gewünschte Winkelposition einnehmen.

Das andere Ende des Flügels 21 weist ebenfalls eine Öffnung auf, die mit 27 bezeichnet ist und für die Hindurchführung und Anbringung eines hier nicht dargestellten Befestigungsbandes vorgesehen ist.

## Patentansprüche

1. Tracheotomiekanüle (100), bestehend aus einem rohr- oder schlauchförmigen Element für die Hindurchführung durch einen Hals- und Luftröhrenschnitt in die Luftröhre eines Patienten, wenn dessen obere Atemwege blockiert sind, um den Patienten durch die Kanüle mit Atemluft zu versorgen, und mit einem an der Kanüle angebrachten Schild (21,22), durch dessen Anlage am Hals des Patienten die Position der Kanüle bzw. des Endes der Kanüle in der Luftröhre des Patienten definiert wird, **dadurch gekennzeichnet, daß** der Schild (21,22) in Längsrichtung der Kanüle in verschiedenen Positionen fixierbar an der Kanüle angebracht ist, daß der Schild an einer Klemmvorrichtung (10) angebracht ist, die mit der Außenseite der Kanüle in lösbarem Klemmeingriff steht und daß die Klemmvorrichtung in Klemmeingriff mit der Kanüle vorgespannt ist.

2. Tracheotomiekanüle nach Anspruch 1, **dadurch gekennzeichnet, daß** die Klemmvorrichtung aus mindestens zwei die Kanüle mindestens teilweise ringförmig umgreifenden Halteteilen (1, 3) besteht, welche relativ zueinander senkrecht zu ihren Ringachsen verschiebbar sind.

3. Tracheotomiekanüle nach Anspruch 2, **dadurch gekennzeichnet, daß** die Halteteile (1, 3) um eine dritte Achse, welche parallel zu den beiden Ringachsen liegt, jedoch nicht mit einer dieser Ringachsen zusammenfällt, verschwenkbar ist.

4. Tracheotomiekanüle nach Anspruch 2, **dadurch gekennzeichnet, daß** eines der Halteteile (3) relativ zu dem anderen (1) linear verschiebbar ist.

5. Tracheotomiekanüle nach Anspruch 4, **dadurch gekennzeichnet, daß** die Klemmvorrichtung im wesentlichen aus drei Teilen besteht, wobei zwei der Teile (1, 2) fest miteinander verbunden sind und ein Gehäuse bilden, welches zwei miteinander fluchtende Öffnungen für das Hindurchführen einer Tracheotomiekanüle aufweist, wobei die Klemmvorrichtung ein drittes, im wesentlichen ebenes Teil (3) aufweist, welches ebenfalls eine Öffnung für die Hindurchführung einer Tracheotomiekanüle hat, die mindestens teilweise mit den beiden miteinander fluchtenden Öffnungen zur Überlappung bringbar ist, wenn das dritte, ebene Teil im Inneren des durch das erste und das zweite Teil gebildeten Gehäuses angeordnet ist, wobei der dritte, ebene Teil derart vorgespannt ist, daß seine Öffnung (13) zu den beiden Öffnungen (11, 12) der anderen Teile (1, 2) exzentrisch verschoben ist, wodurch die Tracheotomiekanüle in dem überlappenden Bereich der drei Öffnungen eingeklemmt wird.

6. Tracheotomiekanüle nach Anspruch 5, **dadurch gekennzeichnet, daß** der dritte, ebene Teil (3) zur Erzeugung der exzentrischen Vorspannung einen Federbügel aufweist, der sich an der Innenwand des von dem ersten und zweiten Teil (1, 2) gebildeten Gehäuses abstützt.

7. Tracheotomiekanüle nach Anspruch 6, **dadurch gekennzeichnet, daß** auf der dem Federbügel (4) gegenüberliegenden Seite des dritten, ebenen Teils (3) ein Betätigungselement (5) vorgesehen ist, weiches sich durch eine in dem Gehäuse freigelassene seitliche Öffnung hindurch nach außen erstreckt, so daß das Betätigungselement (5) von der Gehäuseaußenseite her gegen die Kraft des Federbügels (4) eindrückbar ist, wodurch sich der Überlappungsbereich der drei Öffnungen vergrößert.

8. Tracheotomiekanüle nach einem der Ansprüche 2 bis 7, **dadurch gekennzeichnet, daß** der Innenrand mindestens eines Teils der Öffnungen der Teile der Klemmvorrichtung gezahnt ausgebildet ist.

9. Tracheotomiekanüle nach einem der Ansprüche 1 bis 8, **dadurch gekennzeichnet, daß** der Schild der Tracheotomiekanüle an der Klemmvorrichtung befestigt ist.

10. Tracheotomiekanüle nach Anspruch 9, **dadurch gekennzeichnet, daß** der Schild (21, 22) eine Öffnung aufweist, deren Durchmesser größer ist als der Durchmesser der Tracheotomiekanüle und der auf einen zylindrischen Ansatz der Klemmvorrichtung aufgesetzt ist, wobei der zylindrische Ansatz (6) zu mindestens einer der drei Öffnungen der Klemmvorrichtung im wesentlichen konzentrisch ausgerichtet ist.

11. Tracheotomiekanüle nach Anspruch 10, **dadurch gekennzeichnet, daß** der zylindrische Ansatz (6) an seinem freien Ende einen radial nach außen ragenden Halteflansch (7) aufweist.

12. Tracheotomiekanüle nach einem der Ansprüche 9 bis 11, **dadurch gekennzeichnet, daß** der Schild aus zwei getrennten, relativ zueinander um die Kanüle herum drehbaren Flügeln besteht.

13. Tracheotomiekanüle nach Anspruch 12, **dadurch gekennzeichnet, daß** die relativ zueinander beweglichen Flügel (21, 22) mit jeweils einem Ringabschnitt (23, 24) auf den zylindrischen Ansatz (6) der Klemmvorrichtung aufgesetzt sind.

14. Tracheotomiekanüle nach Anspruch 13, **dadurch gekennzeichnet, daß** die Ringabschnitte (23, 24) auf ihren einander zugewandten Flächen eine Riffelung oder Verzahnung aufweisen, welche ein relatives Verdrehen der Ringelemente behindern.

15. Tracheotomiekanüle nach Anspruch 13 oder 14, **dadurch gekennzeichnet, daß** die Ringabschnitte (23, 24) zwischen dem Flansch (7) und dem Grund des zylindrischen Ansatzes (6) in Eingriff miteinander vorgespannt sind.

16. Tracheotomiekanüle nach einem der Ansprüche 12 bis 15, **dadurch gekennzeichnet, daß** einer der Flügel nicht drehbar an der Klemmvorrichtung fixiert ist.

17. Tracheotomiekanüle nach einem der Ansprüche 9 bis 16, **dadurch gekennzeichnet, daß** der Schild bzw. der zylindrische Ansatz auf der dem Hals eines Patienten zugewandten Seite der Klemmvorrichtung angeordnet sind.

## Claims

1. A tracheotomy cannula (100) comprising an element in tube or hose form for passing through an incision in the neck and trachea into the trachea of a patient if the upper respiratory tracts of the patient are blocked in order to supply the patient with respiration air through the cannula, and with a shield plate (21, 22) which is mounted to the cannula and the contact of which against the neck of the patient defines the position of the cannula or the end of the cannula in the trachea of the patient, **characterised in that** the shield plate (21, 22) is mounted to the cannula fixably in various positions in the longitudinal direction of the cannula, that the shield plate is mounted to a clamping device (10) which is in releasable clamping engagement with the outside of the cannula, and that the clamping device is prestressed into clamping engagement with the cannula.

2. A tracheotomy cannula according to claim 1 **characterised in that** the clamping device comprises at least two holding portions (1, 3) which at least partially embrace the cannula in an annular configuration and which are displaceable relative to each other perpendicularly to their ring axes.

3. A tracheotomy cannula according to claim 2 **characterised in that** the holding portions (1, 3) are pivotable about a third axis which is parallel to the two ring axes but which is not coincident with one of said ring axes.

4. A tracheotomy cannula according to claim 2 **characterised in that** one of the holding portions (3) is linearly displaceable relative to the other (1).

5. A tracheotomy cannula according to claim 4 **characterised in that** the clamping device substantially comprises three portions, wherein two of the portions (1, 2) are fixedly connected to each other and form a housing having two mutually aligned openings for a tracheotomy cannula to pass therethrough, wherein the clamping device has a third, substantially flat portion (3) also having an opening for the passage therethrough of a tracheotomy cannula, which can be at least partially brought into overlapping relationship with the two mutually aligned openings when the third flat portion is arranged in the interior of the housing formed by the first and the second portions, wherein the third flat portion is prestressed in such a way that its opening (13) is eccentrically displaced with respect to the two openings (11, 12) of the other portions (1, 2), whereby the tracheotomy cannula is clamped in the overlapping region of the three openings.

6. A tracheotomy cannula according to claim 5 **characterised in that** to produce the eccentric prestressing effect the third flat portion (3) has a spring bow which bears against the inside wall of the housing formed by the first and second portions (1, 2).

7. A tracheotomy cannula according to claim 6 **characterised in that** provided on the side of the third flat portion (3), which is in opposite relationship to the spring bow (4), is an actuating element (5) which extends outwardly through a lateral opening left free in the housing, so that the actuating element (5) can be pressed in from the outside of the housing against the force of the spring bow (4), whereby the overlap region of the three openings is increased in size.

8. A tracheotomy cannula according to one of claims 2 to 7 **characterised in that** the inside edge of at least a part of the openings of the portions of the clamping device is of a toothed configuration.

9. A tracheotomy cannula according to one of claims 1 to 8 **characterised in that** the shield plate of the tracheotomy cannula is secured to the clamping device.

10. A tracheotomy cannula according to claim 9 **characterised in that** the shield plate (21, 22) has an opening whose diameter is larger than the diameter of the tracheotomy cannula and which is fitted on to a cylindrical projection of the clamping device, wherein the cylindrical projection (6) is oriented substantially concentrically with respect to at least one of the three openings of the clamping device.

11. A tracheotomy cannula according to claim 10 **characterised in that** at its free end the cylindrical projection (6) has a radially outwardly projecting holding flange (7).

12. A tracheotomy cannula according to one of claims 9 to 11 **characterised in that** the shield plate comprises two separate wings which are rotatable relative to each other about the cannula.

13. A tracheotomy cannula according to claim 12 **characterised in that** the wings (21, 22) which are movable relative to each other are fitted with a respective ring portion (23, 24) on to the cylindrical projection (6) of the clamping device.

14. A tracheotomy cannula according to claim 13 **characterised in that** on their mutually facing surfaces the ring portions (23, 24) have a grooving or toothed configuration which prevent relative rotary movement of the ring elements. in

15. A tracheotomy cannula according to claim 13 or claim 14 **characterised in that** the ring portions (23, 24) are prestressed into engagement with each other between the flange (7) and the base of the cylindrical projection (6).

16. A tracheotomy cannula according to one of claims 12 to 15 **characterised in that** one of the wings is non-rotatably fixed to the clamping device.

17. A tracheotomy cannula according to one of claims 9 to 16 **characterised in that** the shield plate or the cylindrical projection respectively are arranged on the side of the clamping device, which is towards the neck of a patient.

## Revendications

1. Canule de trachéotomie (100), se composant d'un élément en forme de tube ou de tuyau destiné passer dans la trachée d'un patient par une incision faite dans la trachée au niveau du cou lorsque les voies respiratoires supérieures du patient sont bloquées afin d'alimenter le patient en air de respiration via la canule, et d'un écran (21, 22), appliqué sur la canule, dont la mise en place au niveau du cou du patient permet de définir la position de la canule resp. de l'extrémité de la canule dans la trachée du patient, **caractérisée en ce que** l'écran (21, 22) est appliqué sur la canule de façon à pouvoir être fixé dans différentes positions dans la direction longitudinale de la canule, l'écran est appliqué sur un dispositif de serrage (10) qui est en engagement par serrage amovible avec le côté extérieur de la canule, et le dispositif de serrage est précontraint en engagement par serrage avec la canule.

2. Canule de trachéotomie selon la revendication 1, **caractérisée en ce que** le dispositif de serrage se compose d'au moins deux pièces de support (1, 3) entourant au moins partiellement la canule à la manière de bagues, lesquelles pièces de support sont déplaçables l'une par rapport à l'autre et perpendiculairement à leur axe de bague.

3. Canule de trachéotomie selon la revendication 2, **caractérisée en ce que** les pièces de support (1, 3) sont aptes à pivoter autour d'un troisième axe qui est parallèle au deux axes de bague sans coïncider cependant avec l'un de ces axes de bague.

4. Canule de trachéotomie selon la revendication 2, **caractérisée en ce que** l'une des pièces de support (3) est déplaçable linéairement par rapport à l'autre (1 ).

5. Canule de trachéotomie selon la revendication 4, **caractérisée en ce que** le dispositif de serrage se compose essentiellement de trois pièces, de sorte que deux des pièces (1, 2) sont reliées fixes l'une à l'autre et forment un boîtier qui comporte deux ouvertures alignées et destinées au passage d'une canule de trachéotomie, le dispositif de serrage comporte une troisième pièce (3) sensiblement plane qui comporte également une ouverture destiné au passage d'une canule de trachéotomie qui peut être amenée au moins partiellement à recouvrement avec les deux ouvertures alignées lorsque la troisième pièce plane est disposée à l'intérieur du boîtier formé par les première et deuxième pièces, et la troisième pièce plane est précontrainte de telle sorte que son ouverture (13) est déplacée excentriquement par rapport aux deux ouvertures (11, 12) des autres pièces (1, 2) et que la canule de trachéotomie est coincée dans la région de recouvrement des trois ouvertures.

6. Canule de trachéotomie selon la revendication 5, **caractérisée en ce que** la troisième pièce plane (3) comporte un étrier élastique en vue de générer la précontrainte excentrique, lequel étrier élastique s'appuie sur la paroi intérieure du boîtier formé par les première et deuxième pièces (1, 2).

7. Canule de trachéotomie selon la revendication 6, **caractérisée en ce qu'**il est prévu, du côté de la troisième pièce plane (3) qui se trouve en face de l'étrier élastique (4), un élément d'actionnement (5) qui s'étend vers l'extérieur par une ouverture latérale ménagée dans le boîtier de sorte que l'élément d'actionnement (5) peut être enfoncé depuis le côté extérieur du boîtier en s'opposant à la force exercée par l'étrier élastique (4) de façon à augmenter la région de recouvrement des trois ouvertures.

8. Canule de trachéotomie selon l'une des revendications 2 à 7, **caractérisée en ce que** le bord intérieur d'au moins une partie des ouvertures ménagées dans les pièces du dispositif de serrage est dentelé.

9. Canule de trachéotomie selon l'une des revendications 1 à 8, **caractérisée en ce que** l'écran de la canule de trachéotomie est fixé au dispositif de serrage.

10. Canule de trachéotomie selon la revendication 9, **caractérisée en ce que** l'écran (21, 22) comporte une ouverture, dont le diamètre est supérieur au diamètre de la canule de trachéotomie, et est placé sur une partie saillante du dispositif de serrage et la partie saillante (6) est dirigée sensiblement concentriquement à au moins l'une des trois ouvertures du dispositif de serrage.

11. Canule de trachéotomie selon la revendication 10, **caractérisée en ce que** la partie saillante cylindrique (6) comporte à son extrémité libre une collerette de retenue (7) saillant radialement vers l'extérieur.

12. Canule de trachéotomie selon l'une des revendications 9 à 11, **caractérisée en ce que** l'écran se compose de deux pattes séparées aptes à tourner l'une par rapport à l'autre autour de la canule.

13. Canule de trachéotomie selon la revendication 12, **caractérisée en ce que** les pattes (21, 22) mobiles l'une par rapport à l'autre comportent chacune une portion annulaire (23, 24) qui vient se placer sur la partie saillante cylindrique (6) du dispositif de serrage.

14. Canule de trachéotomie selon la revendication 13, **caractérisée en ce que** les portions annulaires (23, 24) comporte sur leurs faces dirigées l'une vers l'autre des stries ou bien un endentement qui empêche les éléments annulaires de tourner les uns par rapport aux autres.

15. Canule de trachéotomie selon la revendication 13 ou 14, **caractérisée en ce que** les portions annulaires (23, 24) entre la collerette (7) et le fond de la partie saillante cylindrique (6) sont précontraintes en engagement l'une avec l'autre.

16. Canule de trachéotomie selon l'une des revendications 12 à 15, **caractérisée en ce que** l'une des pattes est fixée immobile en rotation au dispositif de serrage.

17. Canule de trachéotomie selon l'une des revendications 9 à 16, **caractérisée en ce que** l'écran resp. la partie saillante cylindrique est disposé du côté du dispositif de serrage qui est dirigé vers le cou du patient.
